(19) Europäisches Patentamt
European Patent Office
Office européen des brevets

(11) Veröffentlichungsnummer: **0 043 118**
**A1**

(12) # EUROPÄISCHE PATENTANMELDUNG

(21) Anmeldenummer: **81104949.3**

(22) Anmeldetag: **26.06.81**

(51) Int. Cl.³: **C 07 C 63/38**
C 07 C 51/08

(30) Priorität: **28.06.80 DE 3024527**

(43) Veröffentlichungstag der Anmeldung:
**06.01.82 Patentblatt 82/1**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt/Main 80(DE)**

(72) Erfinder: **Frischkorn, Hans, Dr.**
**Martin-Wohmann-Strasse 3**
**D-6238 Hofheim am Taunus(DE)**

(72) Erfinder: **Schinzel, Erich, Dr.**
**Ostpreussenstrasse 43**
**D-6238 Hofheim am Taunus(DE)**

(54) **Verfahren zur Herstellung von Naphthalindicarbonsäure-(1,4).**

(57) Verfahren zur Herstellung von Naphthalindicarbonsäure-(1,4), dadurch gekennzeichnet, daß man 1,4-Dihalogennaphthalin in einem polaren, aprotischen Lösungsmittel mit mindestens 2 Mol Kupfer-(I)-cyanid pro Mol Dihalogennaphthalin umsetzt und den so erhaltenen Kupfersalzkomplex des 1,4-Dicyannaphthalins alkalisch verseift.

EP 0 043 118 A1

Croydon Printing Company Ltd.

## Verfahren zur Herstellung von Naphthalindicarbonsäure-(1,4)

Es ist bereits bekannt, Naphthalindicarbonsäure-(1,4) durch Verseifung des 1,4-Dicyannaphthalins herzustellen /¯vgl. R. Scholl u. H. Neumann, Ber. 55 (1922), 121 und E.F. Bradbrook u. R.P. Linstead, J.chem.Soc. (London) 1936, 1739-1744_7. Die dort beschriebenen Verfahren zur Herstellung des Dinitrils gehen aus von geeigneten Naphthalinsulfonsäuren, welche durch Kaliumcyanidschmelze in das 1,4-Dicyannaphthalin überführt werden. Diese Verfahren erfordern aber extreme Bedingungen, führen in größerem Maße zu dunkelgefärbten Nebenprodukten und sind somit für eine technische Gewinnung der Naphthalindicarbonsäure-(1,4) wenig geeignet.

Es wurde nun gefunden, daß sich Naphthalindicarbonsäure-(1,4) in hoher Reinheit und in technisch gut durchführbarer Weise herstellen läßt, wenn man 1,4-Dihalogennaphthalin in polaren aprotischen Lösungsmitteln mit mindestens 2 Mol Kupfer-(I)-cyanid pro Mol Dihalogennaphthalin, gegebenenfalls in Gegenwart von katalytischen Mengen Kaliumjodid und Kupfersulfat umsetzt und den so erhaltenen Kupfersalzkomplex des 1,4-Dicyannaphthalins alkalisch verseift.

Als Dihalogennaphthaline eignen sich das in der Literatur schon länger beschriebene 1,4-Dichlornaphthalin (vgl. DRP 286 489) und das ebenfalls schon länger bekannte 1,4-Dibromnaphthalin (vgl. J.S. Salkind und S.B. Faerman, Journal der russischen physikalischen Gesellschaft, Chemischer Teil 62 (1930), 1021-1032). Beide Dihalogennaphthaline sind in einfacher Weise aus dem technisch gut zugänglichen Grundkörper Naphthalin herzustellen. Die angewendete Menge des Kupfer-(I)-cyanids schwankt zwischen 2 und 2,5 Mol, bezogen auf 1 Mol der Dihalogenverbindung. Als polares, aprotisches Lösungsmittel wendet man N-Methylacetamid, Dimethylacetamid, Dimethylsulfoxid, N-Methylpyrrolidon, Hexamethylphosphorsäuretriamid, Chinaldin und vorzugsweise Dimethylformamid und Chinolin an. Im Falle der Dibromverbindung kann vorteilhaft Dimethylformamid verwendet werden. Die träger reagierende Dichlorverbindung wird vorzugsweise in dem höher siedenden Chinolin zur Umsetzung gebracht. Die Reaktion wird bei Temperaturen von 150-250°C durchgeführt, vorteilhaft arbeitet man beim Siedepunkt des eingesetzten Lösungsmittels. Außerdem ist es vorteilhaft, die Reaktion durch Zugabe eines Katalysators zu beschleunigen. Zu diesem Zweck kann man ein Gemisch aus Kaliumjodid und Kupfersulfat nehmen.

Bei dieser Reaktion fällt das 1,4-Dicyannaphthalin als Kupfer-(I)-salzkomplex folgender Zusammensetzung an

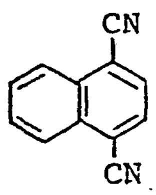 · 2 Cu Hal          Hal = Br, Cl

das nach Abkühlen des Reaktionsgemisches und nach Zusatz von Wasser oder eines niedrig siedenden Lösungsmittels isoliert werden kann. Als niedrig siedendes polares Lösungsmittel werden niedere Alkohole, vorzugsweise Methanol oder Ethanol verwendet.
Ohne das 1,4-Dicyan-naphthalin freizusetzen wird der noch feuchte Kupfersalzkomplex dann nach bekannten Methoden direkt alkalisch verseift mit einem starken Alkali, beispielsweise mit überschüssiger 10-35 %iger Natronlauge in der Siedehitze. Anschließend stellt man den pH-Wert der Lösung auf 7 bis 8 und trennt das dabei quantitativ ausgeschiedene Kupferoxid ab. Aus dem Filtrat wird durch weitere Zugabe von Mineralsäure bis auf pH 2 die Naphthalindicarbonsäure abgeschieden, die abgetrennt wird.

Die Aufarbeitung kann auch in der Weise erfolgen, daß man auf die Isolierung des Kupfersalz-Komplexes des 1,4-Dicyannaphthalins verzichtet und nach Beendigung des Halogen-Cyanid-Austausches das Reaktionsgemisch mit starkem Alkali, wie etwa 10-35 %ige Natronlauge, direkt hydrolisiert.

Verwendet man dabei Chinolin oder Chinaldin als Lösungsmittel, so kann man dies während der Hydrolyse mit Wasserdampf abdestillieren.

Das erfindungsgemäße Verfahren kann auch so durchgeführt werden, daß man von 1,4-Dihalogennaphthalinen ausgeht, die noch mit anderen stellungsisomeren Dihalogennaphthalinen verunreinigt sind und die daraus erhaltene rohe Naphthalindicarbonsäure-(1,4) einer Reinigung unterwirft. Als stellungsisomere Dihalogennaphthaline, die im 1,4-Dihalogennaphthalin enthalten sein können, kommen vor allem die 1,5- und 2,6- wie auch die 2,7-Isomeren in Frage. Zur Reinigung löst man die rohe Naphthalindicarbonsäure-(1,4) nach der Trocknung in einem geeigneten Lösungsmittel und trennt von den schwerlöslichen Isomeren Naphthalindicarbonsäuren-(1,5) und/oder -(2,6) und/oder -(2,7) durch Filtration ab. Für die Reinigungsoperation sind vorwiegend niedrig siedende Glykolmonoether, wie Ethylenglykolmonomethyl- und -monoethylether geeignet.

Die nach dem erfindungsgemäßen Verfahren erhaltene Naphthalindicarbonsäure-(1,4) findet Verwendung als Vorprodukt für Farbstoffe, UV-Absorber, Szintillatoren und optische Aufheller. Sie ist frei von gefärbten Verunreinigungen und kann direkt zur weiteren Synthese eingesetzt werden.

Die folgenden Beispiele erläutern die Erfindung näher. Temperaturen sind in °C angegeben.

Beispiel 1

In 330 Gewichtsteile wasserfreies Chinolin werden 98,6 Gewichtsteile 1,4-Dichlornaphthalin vom Erstarrungspunkt 60°, 100 Gewichtsteile Kupfer-(I)-cyanid (67-71 % Kupfer), 2 Gewichtsteile wasserfreies Kupfersulfat und 2 Gewichtsteile Kaliumjodid eingetragen. Anschließend erhitzt man unter Rühren 20 Stunden am Rückfluß auf 235-240°, wobei eine homogene dunkle Lösung entsteht. Nach Abkühlen auf 120-130° versetzt man das Reaktionsgemisch mit 1125 Gewichtsteilen einer 20 %igen Natronlauge und erhitzt die entstandene gelblich braune Suspension am Rückfluß, wobei der Rücklauf über ein geeignetes Phasentrenngerät geleitet wird und man die spezifisch schwerere Phase - wasserhaltiges Chinolin - abzieht. Nach vollständiger Verseifung des 1,4-Dicyannaphthalin-Kupfersalzkomplexes bzw. vollständiger Abtreibung des Chinolins (nach ca. 6 Stunden) läßt man auf 80° abkühlen, stumpft mit ca. 465 Gewichtsteilen konzentrierter Salzsäure (ca. 31 %ig) auf pH 7-8 ab und saugt vom ausgeschiedenen Kupferoxidschlamm ab, den man noch mit Wasser nachwäscht. Im Filtrat fällt man das Reaktionsprodukt Naphthalindicarbonsäure-(1,4) durch Zugabe von ca. 105 Gewichtsteilen konzentrierter Salzsäure (31 %ig) aus, saugt ab und wäscht mit Wasser chlorionenfrei. Nach dem Trocknen erhält man 87 Gewichtsteile einer praktisch farblosen Dicarbonsäure mit einem Schmelzpunkt von 315-320°.

Beispiel 2

In 305 Gewichtsteile Dimethylformamid werden 143 Gewichtsteile 1,4-Dibromnaphthalin vom Erstarrungspunkt 78° und 100 Gewichtsteile Kupfer-(I)-cyanid (67-71 % Kupfer) eingetragen. Anschließend erhitzt man unter Rühren 4 Stunden am Rückfluß auf 150-160°, wobei anfänglich Lösung eintritt, dann aber nach und nach der 1,4-Dicyannaphthalin-Kupferbromid-Komplex ausfällt. Nach Abkühlen auf ca. 60° verdünnt man das Reaktionsgemisch mit 635 Gewichtsteilen Methanol, rührt ca. eine Stunde bei Raumtemperatur nach, saugt ab und wäscht mit ca. 160 Gewichtsteilen Methanol nach. Der so

isolierte 1,4-Dicyannaphthalin-Kupferbromid-Komplex wird in 680 Gewichtsteile 33 %ige Natronlauge eingetragen und unter Rühren und Abdestillation des eingebrachten Methanols auf 100 bis 105° erhitzt. Anschließend führt man durch 6stündiges Erhitzen am Rückfluß die Hydrolyse durch, kühlt auf ca. 80° und stellt nach Verdünnen mit 335 Gewichtsteilen Wasser mit ca. 460 Gewichtsteilen konzentrierter Salzsäure auf pH 7,0-7,5. Man saugt vom ausgeschiedenen Kupferoxidschlamm ab und wäscht diesen mit ca. 500 Gewichtsteilen Wasser portionsweise nach. Im Filtrat wird durch Zugabe von ca. 130 Gewichtsteilen konzentrierter Salzsäure bei pH 2 die Naphthalindicarbonsäure-(1,4) ausgefällt und anschließend abgesaugt. Man wäscht mit Wasser chlorionenfrei und erhält nach dem Trocknen 90 Gewichtsteile einer praktisch farblosen Säure mit einem Schmelzpunkt von 315-320°.

Beispiel 3

Man verfährt wie unter Beispiel 2, setzt aber 143 Gewichtsteile rohes 1,4-Dibromnaphthalin ein, wie es durch Umsetzung von Brom mit Naphthalin in Eisessig erhalten werden kann. Diese Rohware hat einen Erstarrungspunkt von 62-64° und enthält bis zu 25 % des 1,5 Isomeren. Man erhält nach dem Trocknen 86 Gewichtsteile einer rohen Naphthalindicarbonsäure-(1,4), die bis zu 20 % der isomeren 1,5-Dicarbonsäure enthalten kann. Zur Reinigung erhitzt man die Rohsäure in 1660 Gewichtsteilen Ethylenglykolmonomethylether ca. eine Stunde zum Sieden, um eine völlige Lösung der Dicarbonsäure-(1,4) zu erhalten, kühlt auf Raumtemperatur ab und saugt nach einstündigem Nachrühren ab. Als Rückstand bleibt eine verunreinigte Naphthalindicarbonsäure-(1,5). Vom Filtrat werden ca. 1400 Gewichtsteile Ethylenglykolmonomethyläther abdestilliert und der Sumpf mit 1400 Gewichtsteilen Wasser versetzt, wobei die Naphthalindicarbonsäure-(1,4) vollständig ausgefällt wird. Man saugt ab und wäscht mit ca. 100 Gewichtsteilen Wasser nach. Nach dem Trocknen erhält man 67 Gewichtsteile einer farblosen Naphthalindicarbonsäure-(1,4) mit einem Schmelzpunkt von 315-320°.

Patentansprüche:

1. Verfahren zur Herstellung von Naphthalin-dicarbonsäure-(1,4), dadurch gekennzeichnet, daß man 1,4-Dihalogennaphthalin in einem polaren, aprotischen Lösungsmittel mit mindestens 2 Mol Kupfer-(I)-cyanid pro Mol Dihalogennaphthalin umsetzt und den so erhaltenen Kupfersalzkomplex des 1,4-Dicyannaphthalins alkalisch verseift.

2. Verfahren nach Anspruch 1, dadurch gekennzeichnet, daß man den Kupfersalzkomplex des 1,4-Dicyannaphthalins durch Zugabe von Wasser oder einem niedrig siedenden polaren Lösungsmittel ausfällt, abtrennt und verseift.

3. Verfahren nach Anspruch 1, dadurch gekennzeichnet, daß man den Kupfersalzkomplex des 1,4-Dicyananphthalins nach dem Cyanidaustausch direkt ohne vorherige Isolierung in dem Reaktionsgemisch hydrolisiert.

0043118

Europäisches Patentamt

# EUROPÄISCHER RECHERCHENBERICHT

Nummer der Anmeldung

EP 81 10 4949.3

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| A | JOURNAL OF ORGANIC CHEMISTRY, Band 41, Nr. 6, 1976<br>Columbus<br>J.M. McNAMARA et al. "Synthesis of 4-Cyano-4'-halobiphenyls"<br>Seite 1071<br>-- | 1 | C 07 C 63/38<br>C 07 C 51/08 |
| | US - A - 2 195 076 (W. BRAUN et al.)<br>* Anspruch 1; Beispiel 14 *<br>-- | 1 | |
| A | DE - A - 2 249 880 (THE LUMMUS)<br>* Ansprüche 1, 7 *<br>-- | 1 | RECHERCHIERTE SACHGEBIETE (Int. Cl.3)<br>C 07 C 51/00<br>C 07 C 51/08<br>C 07 C 63/38<br>C 07 C 121/62 |
| A | DE - A - 2 417 362 (SUN VENTURES)<br>* Anspruch 1 *<br>-- | 1 | |
| | JOURNAL OF ORGANIC CHEMISTRY, Band 34, Nr. 11, 1969<br>Columbus<br>H.O. HOUSE et al. "Reaction of Sodium Dicyanocuprate with Vinyl and Aryl Halides"<br>Seiten 3626 bis 3627<br>---- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 29-09-1981 | KNAACK |